Europäisches Patentamt

**European Patent Office**

**Office européen des brevets**

(11) Numéro de publication: **0 114 145**
A2

## (12) DEMANDE DE BREVET EUROPEEN

(21) Numéro de dépôt: **84420007.1**

(22) Date de dépôt: **10.01.84**

(51) Int. Cl.³: **A 61 M 5/20, A 61 M 5/28**

(30) Priorité: **17.01.83 FR 8300891**

(71) Demandeur: **Brunet, Jean-Louis, 14, rue Victor Hugo, F-69002 Lyon (FR)**

(43) Date de publication de la demande: **25.07.84 Bulletin 84/30**

(72) Inventeur: **Brunet, Jean-Louis, 14, rue Victor Hugo, F-69002 Lyon (FR)**

(84) Etats contractants désignés: **AT BE CH DE FR GB IT LI LU NL SE**

(74) Mandataire: **Maureau, Philippe, Cabinet Germain & Maureau Le Britannia - Tour C 20, bld Eugène Déruelle, F-69392 Lyon Cédex 03 (FR)**

(54) **Seringue à usage médical.**

(57) Cette seringue est du type comprenant une ampoule (16) contenant le liquide (7) à injecter, équipée à son extrémité avant de l'aiguille d'injection (8), et obturée à son extrémité postérieure par piston dont le déplacement provoque l'injection de liquide.

Selon l'invention, l'organe (20) de maintien en position et d'obturation de l'aiguille avant utilisation est réalisé en un matériau différent de celui constitutif du corps de l'ampoule.

Application aux seringues à injection automatique.

EP 0 114 145 A2

ACTORUM AG

1
## "SERINGUE A USAGE MEDICAL"

La présente invention a pour objet une seringue à usage médical et plus spécialement une seringue automatique permettant la réalisation d'une injection sous-cutanée, en urgence, par le patient lui-même ou par une personne ne possédant pas de qualification médicale.

Certaines personnes sujettes à des allergies, souffrant de maladies cardio-vasculaires ou de certaines formes de diabète, peuvent présenter des troubles se manifestant par un état de choc qu'il convient de traiter immédiatement.

C'est ainsi que les hypersensibilités entraînant des réactions anaphylactiques doivent être traitées par injection d'adrénaline.

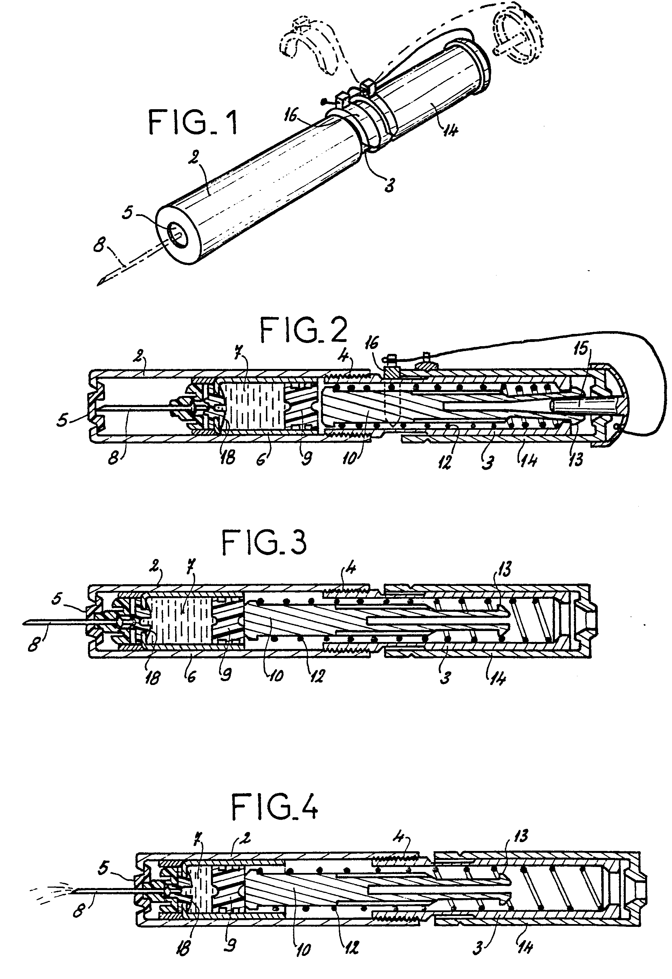

Pour les traitements d'urgence, il est connu d'utiliser une seringue d'auto-injection du type de celle représentée aux figures 1 à 4 du dessin schématique annexé.

Cette seringue contient un boîtier cylindrique en deux parties, c'est-à-dire une partie avant (2) et une partie arrière (3) fixées l'une sur l'autre par vissage en (4). La partie avant (2) présente dans sa paroi d'extrémité une ouverture fermée par un obturateur (5) en élastomère, facilement perforable.

La partie avant (2) du boîtier sert au logement d'une ampoule (6) contenant le liquide (7) de traitement, équipée à son extrémité avant d'une aiguille (8) et obturée à son extrémité arrière par un piston coulissant (9).

Il ressort de la figure 2 qu'en position de repos l'ampoule est disposée dans la zone postérieure de la partie (2) du boîtier et que l'aiguille (8) est située à l'intérieur du boîtier.

La partie arrière (3) du boîtier contient un piston (10) poussé élastiquement vers l'avant par un ressort (12), avec possibilité de maintien en position reculée par l'intermédiaire de crochets (13) associés à son extrémité postérieure, venant prendre appui sur l'extrémité postérieure de la partie arrière (3) du boîtier.

Une douille est montée coulissante sur la partie arrière (3) du boîtier, qui est susceptible de libérer les crochets (13), laissant le ressort (12) se détendre pour assurer un déplacement vers l'avant d'un piston de percussion (10) qui, agissant sur le piston (9) de l'ampoule (6), assure dans un premier temps, et comme représenté à la figure 3, le transfert vers l'avant de l'ensemble ampoule (6) et aiguille (8), avec perforation de l'obturateur (5), puis, dans un second temps, le déplacement du piston (9) à l'intérieur de l'ampoule (6), provoquant l'évacuation du liquide hors de l'ampoule par l'intermédiaire de l'aiguille (8). Il est clair que, préalablement à l'action sur la douille (14), la seringue est positionnée relativement au corps du patient, de telle sorte que le déplacement de l'aiguille (8) se fasse avec mise en position sous-cutanée de celle-ci, immédiatement avant l'injection.

Afin d'éviter toute manoeuvre accidentelle de la seringue pouvant être dangereuse, il est prévu une goupille de sécurité (15), destinée à être engagée entre les crochets (13), de façon à éviter le rapprochement de ceux-ci et par suite la libération du piston (10), et un anneau ouvert (16), destiné à venir coiffer la zone dégagée de la partie postérieure (3) du boîtier, afin d'éviter un coulissement de la douille (14).

La seringue traditionnelle contient une ampoule (6) réalisée en matière synthétique, dont l'extrémité avant est conformée de façon à constituer un porte-aiguille.

Néanmoins, ce type d'ampoule ne convient pas pour un certain nombre de liquides, et notamment pour l'adrénaline qui est un produit particulièrement intéressant à utiliser en urgence dans des seringues à injection automatique.

La présente invention vise à remédier à ces inconvénients.

A cet effet, dans la seringue qu'elle concerne, l'organe de maintien en position et d'obturation de l'aiguille avant utilisation est réalisé en matériau différent de celui constitutif du corps de l'ampoule.

Il est ainsi possible de réaliser le corps de l'ampoule en verre, qui présente l'intérêt d'être compatible avec tous les liquides médicaux, et de réaliser l'organe de positionnement et d'obturation de l'aiguille en une autre matière, telle qu'une matière synthétique. Cette amélioration élargit considérablement le champ d'application des seringues à injection automatique.

De toute façon, l'invention sera bien comprise à l'aide de la description qui suit en référence au dessin schématique annexé, représentant, à titre d'exemples non limitatifs, plusieurs formes d'exécution de cette seringue :

Figures 1 à 4 sont quatre vues d'une seringue équipée d'un premier type d'ampoule, respectivement en perspective et en coupe longitudinale dans trois positions différentes ;

Figure 5 est une vue en coupe longitudinale et à échelle agrandie de l'ampoule de la seringue de figure 1 ;

Figures 6 et 7 sont deux vues en coupe longitudinale de l'extrémité avant d'une autre seringue d'auto-injection, respectivement en période de stockage et en période d'injection ;

Figures 8 et 9 sont deux vues en coupe longitudinale d'une troisième forme d'exécution de l'extrémité avant d'une seringue d'auto-injection, respectivement en période de stockage et en période d'injection ;

Figure 10 et 11 sont deux vues en coupe longitudinale d'une quatrième forme d'exécution de l'extrémité avant d'une seringue d'auto-injection, respectivement en période de stockage et en période d'injection ;

Figure 12 est une vue en coupe longitudinale d'une variante de réalisation de l'ampoule représentée en figure 5.

Dans la forme d'exécution représentée aux figures 1 à 4, la seringue est équipée d'une ampoule (6) en verre, de forme générale cylindrique, dont l'extrémité postérieure est obturée par un piston (9) en élastomère siliconé, et dont l'extrémité antérieure présente une ouverture (17) de diamètre inférieur à celui du corps. Cette ouverture (17) est obturée par un bouchon (18) à double lèvre, réalisé en élastomère semi-souple. Ce joint présente une section en H, dont l'âme est engagée dans l'ouverture (17), dont l'aile (19) prend appui sur la face intérieure du fond de l'ampoule, et dont l'aile extérieure (20) formant un disque prend appui sur la paroi extérieure de l'extrémité de l'ampoule, cette aile (20) servant au support d'une douille cylindrique (22) réalisée par exemple en polypropylène, dans laquelle est engagé le porte-aiguille (23), qui peut être pour sa part réalisé en polyéthylène.

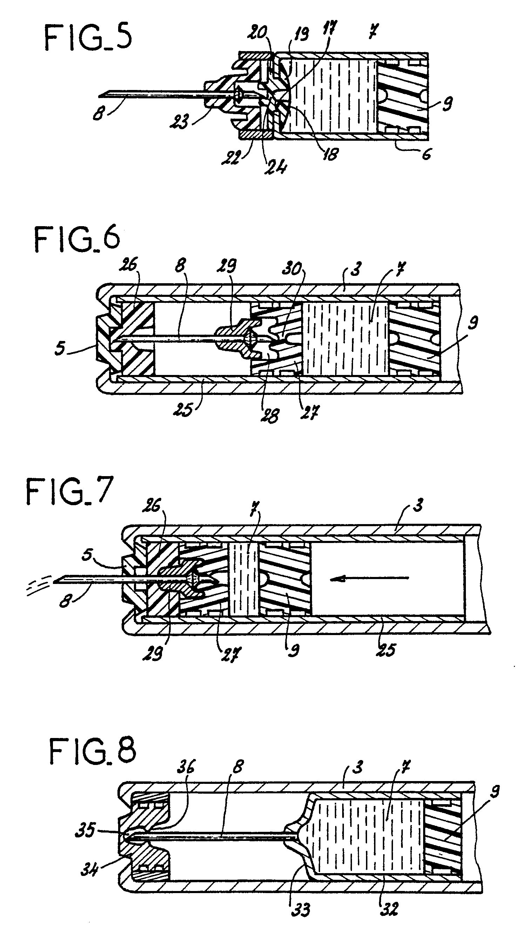

Comme montré notamment à la figure 5, la partie centrale (24) du bouchon (18) est de très faible épaisseur, de façon à pouvoir être perforée par l'extrémité postérieure de l'aiguille (8) lors de la percussion de celle-ci contre l'obturateur (5) de la partie avant (2) du boîtier.

Les figures 6 et 7 représentent une variante de réalisation de cette seringue dans laquelle l'ampoule est constituée par un élément purement cylindrique (25) en verre, de longueur sensiblement supérieure à celle nécessaire pour l'obtention du volume utile de l'ampoule.

4

Dans le cas présent, l'élément cylindrique (25) est monté dans la partie avant du boîtier (3), sans possibilité de coulissement à l'intérieur de celle-ci. L'élément cylindrique (25) est obturé à son extrémité postérieure par un piston (9) de type connu, défini précédemment, et à son extrémité avant par un organe (26) en appui contre l'obturateur (5) servant au guidage de l'aiguille (8) lors du déplacement de celle-ci.

Le volume utile de l'ampoule est délimité à son extrémité avant par un piston (27) en élastomère siliconé de façon à parfaitement coulisser dans l'élément cylindrique (25), dont l'extrémité avant forme un évidement cylindrique (28) dans lequel est engagé l'organe (29) formant porte-aiguille. La partie centrale (30) du piston (27) est amincie et facilement perforable, comme dans le cas précédent, de façon à pouvoir être traversée par l'extrémité postérieure de l'aiguille (8).

D'un point de vue pratique, lorsque le piston de percussion (10) exerce une pression sur le piston (9) contenu dans l'ampoule (25), celui-ci se déplace vers l'avant, provoquant, du fait de l'incompressibilité du liquide (7) contenu dans l'ampoule, le déplacement vers l'avant du piston (27) et de l'aiguille (8) qui, au cours de ce mouvement, perfore l'obturateur (5).

Lorsque le piston avant (27) vient en contact contre la butée (26), l'aiguille (8) le perfore et, le piston (9) poursuivant sa course, le liquide (7) est évacué de l'ampoule, comme montré à la figure 7.

Pour des raisons de simplicité, le piston avant (27) peut être identique au piston arrière (9).

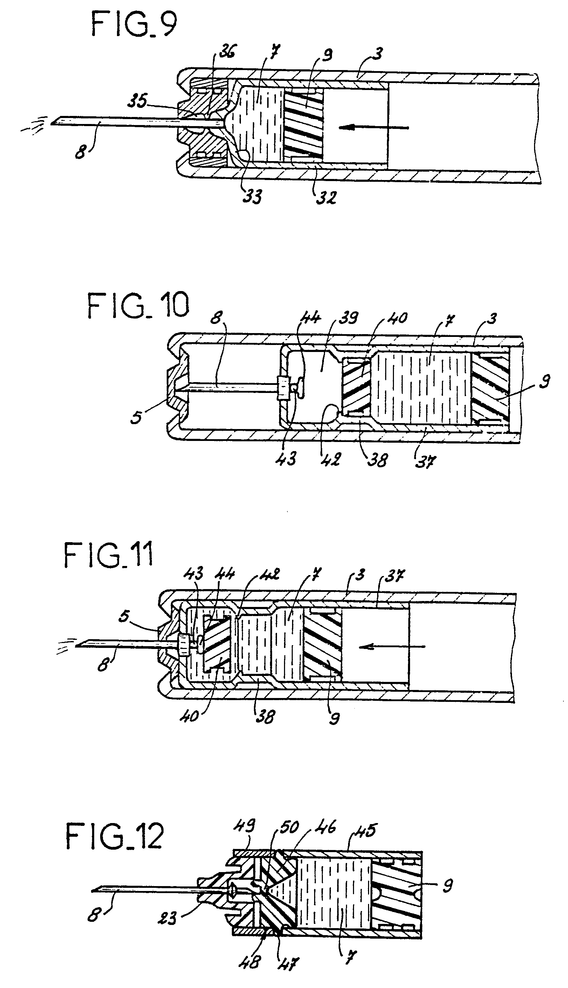

Dans la forme d'exécution représentée aux figures 8 et 9, l'ampoule (32) est de forme cylindrique, étant obturée à son extrémité postérieure par un piston (9) connu, et possédant une extrémité antérieure (33) conformée de façon à assurer la tenue avec étanchéité de l'aiguille (8).

L'obturateur (34) de l'extrémité avant du boîtier (3) dans lequel est monté l'ampoule (32) est conformé de manière à ménager un logement (35) dans lequel est située l'extrémité avant de l'aiguille (8), ce logement étant fermé avec étanchéité en raison de la présence d'une partie rétrécie (36) prenant appui avec étanchéité sur le contour de l'aiguille (8).

Ceci permet d'assurer une bonne étanchéité de l'intérieur de l'ampoule (32) vis-à-vis du milieu extérieur, malgré sa communication permanente avec l'aiguille (8).

D'un point de vue pratique, la poussée exercée par le piston percuteur (10) sur le piston (9) se traduit dans un premier temps par le déplacement de l'ampoule (32) dans le boîtier (3) avec perforation de l'obturateur (34) puis, lorsque l'ampoule vient en butée à l'extrémité avant du boîtier (3), par déplacement du piston (9) à l'intérieur de l'ampoule (32), ce qui se traduit par l'évacuation du liquide (7) hors de l'ampoule.

Dans la forme d'exécution représentée aux figures 10 et 11, l'aiguille (8) est fixée avec étanchéité à l'extrémité avant de l'ampoule (37) dans laquelle elle débouche, cette ampoule présentant un rétreint (38) séparant le volume utile de celle-ci contenant le liquide (7) de sa partie avant (39) dans laquelle débouche l'aiguille. Ce rétreint (38) sert au logement d'un bouchon (40) maintenu en position par un épaulement (42), situé à l'extrémité avant du rétreint (38).

L'orifice (43) de mise en communication de l'aiguille avec le volume avant (39) de l'ampoule (37) est disposé latéralement, l'extrémité de l'aiguille étant équipée d'une butée (44) protégeant celle-ci vis-à-vis du bouchon (40).

D'un point de vue pratique, la pression exercée par le piston de percussion (10) sur le piston (9) obturant l'extrémité postérieure de l'ampoule (37) se traduit par le déplacement de l'ampoule jusqu'à ce qu'elle vienne en butée contre l'extrémité avant du boîtier (3). La pression exercée par le piston (9) sur le liquide (7) provoque alors le transfert du bouchon (40) dans la chambre (39), permettant l'évacuation du liquide par l'aiguille hors de l'ampoule (37).

La figure 12 correspond à une variante d'exécution de l'ampoule de la figure 5, dans laquelle l'ampoule (45) est constituée par un élément cylindrique en verre de section constante, dont l'extrémité postérieure est obturée par un piston (9) traditionnel, et dont l'extrémité avant est obturée par un bouchon (46), dont une extrémité engagée à l'intérieur du corps est limitée par un épaulement périphérique (47) venant en appui contre le bord avant de l'ampoule. Ce bouchon est prolongé à l'extérieur par une partie de diamètre inférieur du corps de l'ampoule formant une portée (48) pour une douille (49) à l'intérieur de laquelle est engagé le porte-aiguille (23).

Comme dans les cas précédents, la partie centrale (50) du bouchon (46) est mince, de manière à pouvoir être perforée facilement par l'aiguille (8).

7

## REVENDICATIONS

1. - Seringue à usage médical, du type comprenant une ampoule (6, 25, 32, 37, 45) contenant le liquide (7) à injecter, équipée à son extrémité avant d'une aiguille d'injection (8), et obturée à son extrémité postérieure par un piston (9) dont le déplacement provoque, dans un premier temps, le déplacement de l'aiguille et la pénétration de celle-ci dans la zone d'injection, et dans un second temps, l'injection de liquide, caractérisée en ce qu'elle comprend un organe (20, 34, 49) qui, assurant le maintien en position et l'obturation de l'aiguille avant utilisation, est réalisé en un matériau différent de celui constitutif du corps de l'ampoule.

2. - Seringue selon la revendication 1, caractérisée en ce que le corps de l'ampoule est réalisé en verre, tandis que l'organe de maintien et d'obturation de l'aiguille est réalisé en un matériau différent.

3. - Seringue selon l'une quelconque des revendications 1 et 2, caractérisée en ce que l'ampoule (6) comprend un corps de forme générale cylindrique dont l'extrémité antérieure présente une ouverture centrale (17) de diamètre inférieur à celui du corps, obturé par un bouchon (18) qui, réalisé en une matière semi-souple compatible avec le liquide contenu dans l'ampoule, forme un joint à double lèvre présentant une section en H, dont les ailes sont en appui sur les faces intérieure et extérieure du fond de l'ampoule, et dont l'âme de faible épaisseur est susceptible d'être perforée par l'aiguille, l'aile (20) située à l'extérieur de l'ampoule formant un disque sur lequel est engagée une douille (22) à l'intérieur de laquelle est emboîté l'organe porte-aiguille (23), de telle sorte que l'extrémité postérieure de l'aiguille se trouve à proximité de la partie centrale du bouchon.

4. - Seringue selon l'une quelconque des revendications 1 et 2, caractérisée en ce que le corps de l'ampoule comprend un corps (45) de forme générale cylindrique de section constante, dont l'extrémité avant est obturée par un bouchon (46) dont une partie, engagée à l'intérieur du corps, est limitée par un épaulement périphérique (48) destiné à venir en appui contre le bord avant de l'ampoule, ce bouchon étant prolongé, à l'extérieur de l'ampoule, par une partie (48) de diamètre inférieur au diamètre extérieur du corps de l'ampoule, sur lequel est engagée une douille (49) à l'intérieur de laquelle est emboîté l'organe porte-aiguille (23), de telle sorte que l'extrémité postérieure de l'aiguille

8

se trouve à proximité de la partie centrale (50), facilement perforable, du bouchon.

5. - Seringue selon l'une quelconque des revendications 1 et 2, caractérisée en ce que l'ampoule comprend un corps (25) de forme générale cylindrique, ouvert à ses deux extrémités, et de longueur sensiblement égale à celle nécessaire à l'obtention du volume utile de l'ampoule, augmentée de la longueur de l'aiguille, l'extrémité du volume utile de l'ampoule étant obturée par un bouchon (27) en forme de piston, dont la zone centrale (30) est perforable, et dont la partie avant forme un évidement cylindrique (28) dans lequel est engagé l'organe porte-aiguille (29).

6. - Seringue selon la revendication 5, caractérisée en ce que le piston (27), délimitant l'extrémité avant du volume utile de l'ampoule, est identique au piston (9) délimitant l'extrémité arrière de celui-ci.

7. - Seringue selon l'une quelconque des revendications 1 et 2, caractérisée en ce que l'extrémité postérieure de l'aiguille (8) est fixée avec étanchéité à l'extrémité avant de l'ampoule (32) dans laquelle elle débouche, tandis que l'extrémité avant de l'aiguille est enfermée dans un logement (35) ménagé dans l'obturateur perforable (34) bouchant l'extrémité avant du corps tubulaire (3) dans laquelle est montée l'ampoule.

8. - Seringue selon l'une quelconque des revendications 1 et 2, caractérisée en ce que l'aiguille (8) est fixée avec étanchéité à l'extrémité avant de l'ampoule (37) dans laquelle elle débouche, l'ampoule présentant un rétreint (38) qui, séparant le volume utile de celle-ci contenant le liquide de sa partie avant (39) dans laquelle débouche l'aiguille, sert au logement d'un bouchon (40) susceptible d'être transféré dans la partie avant de l'ampoule de façon à mettre celle-ci en communication avec le volume utile, sous l'action d'une pression de valeur prédéterminée.

9. - Seringue selon la revendication 8, caractérisée en ce que l'extrémité avant du rétreint (38) de l'ampoule (37) est limitée par un épaulement (42) faisant saillie vers l'intérieur.

10. - Seringue selon l'une quelconque des revendications 8 et 9, caractérisée en ce que la partie avant (39) de l'ampoule (37) dans laquelle débouche l'aiguille (8) est équipée d'un organe (44) protégeant l'extrémité de celle-ci.

0114145

FIG.1

FIG.2

FIG.3

FIG.4

0114145

FIG.5

FIG.6

FIG.7

FIG.8

0114145

FIG.9

FIG.10

FIG.11

FIG.12